# EUROPÄISCHE PATENTSCHRIFT

(11) **EP 1 414 446 B1**
(45) Veröffentlichungstag und Bekanntmachung des Hinweises auf die Patenterteilung: **19.10.2005**
(21) Anmeldenummer: 02767273.2
(22) Anmeldetag: 30.07.2002
(51) Int. Cl.: A61K 31/425

(54) **PRAMIPEXOL ALS ANTIKONVULSIVUM**
PRAMIPEXOL AS AN ANTICONVULSIVE AGENT
UTILISATION DU PRAMIPEXOL COMME ANTICONVULSIF

(30) Priorität: 02.08.2001 DE 10137453
(43) Veröffentlichungstag der Anmeldung: 06.05.2004
(73) Patentinhaber: Boehringer Ingelheim Pharma GmbH & Co.KG, 55216 Ingelheim am Rhein (DE)
(72) Erfinder: CROENLEIN, Jens, 88441 Mittelbiberach (DE)
(86) Internationale Anmeldenummer: PCT/EP2002/008448
(87) Internationale Veröffentlichungsnummer: WO 2003/013519

(56) Entgegenhaltungen:
- WO-A-96/18395
- US-A- 4 886 812

## Beschreibung

Die Erfindung betrifft die Verwendung von 2-Amino-4,5,6,7-tetrahydro-6-n-propylamino-benzothiazol, sein (+) oder (-) Enantiomer, deren pharmakologisch verträgliche Säureadditionssalze sowie Hydrate und Solvate zur Herstellung eines Arzneimittels mit antikonvulsiver Wirkung.

### Hintergrund der Erfindung

Das 2-Amino-6-n-propylamino-4,5,6,7-tetrahydrobenzo-thiazol ist ein Dopaminagonist der auch unter dem Namen Pramipexol im Stand der Technik bekannt ist. Pramipexol, sowie Verfahren zu dessen Herstellung sind beispielsweise aus EP-A-186 087 und US 4,886,812 bekannt. Vor allem die Verwendbarkeit von Pramipexol zur Behandlung der Schizophrenie und insbesondere zur Behandlung des Parkinsons ist bekannt. Der Stand der Technik offenbart beispielsweise ferner, daß Pramipexol den Prolactinserumspiegel senkt (DE 38 43 227).

### Beschreibung der Erfindung

Überraschenderweise wurde gefunden, daß Pramipexol in therapeutisch wirsamen Dosen zur Behandlung cerebraler Anfälle als Antikonvulsivum eingesetzt werden kann.

Dementsprechend betrifft die vorliegende Erfindung die Verwendung von 2-Amino-4,5,6,7-tetrahydro-6-n-propylamino-benzothiazol, gegebenenfalls in Form seines (+) oder seines (-) Enantiomers, gegebenenfalls in Form der pharmakologisch verträgliche Säureadditionssalze sowie Hydrate und Solvate zur Herstellung eines Arzneimittels mit antikonvulsiver Wirkung.

Die vorliegende Erfindung betrifft ferner die Verwendung von 2-Amino-4,5,6,7-tetrahydro-6-n-propylamino-benzothiazol, gegebenenfalls in Form seines (+) oder seines (-) Enantiomers, gegebenenfalls in Form der pharmakologisch verträgliche Säureadditionssalze sowie Hydrate und Solvate zur Herstellung eines Arzneimittels zur Vorbeugung und/oder Behandlung cerebraler Anfälle.

Im Rahmen der vorliegenden Erfindung ist cerebraler Anfall als gleichbedeutend mit cerebralem Krampfanfall zu verstehen.

Ferner betrifft die vorliegende Erfindung die Verwendung von 2-Amino-4,5,6,7-tetrahydro-6-n-propylamino-benzothiazol, gegebenenfalls in Form seines (+) oder seines (-) Enantiomers, gegebenenfalls in Form der pharmakologisch verträgliche Säureadditionssalze sowie Hydrate und Solvate zur Herstellung eines Arzneimittels zur Vorbeugung und/oder Behandlung generalisierter Anfälle (Absencen, auch atypische Absencen, myoklonische, klonische, tonische und tonisch-klonische Anfälle), fokaler (einfach und komplex fokale) und sekundär generalisierter Anfälle.

Besonders bevorzugt betrifft die vorliegende Erfindung die Verwendung von 2-Amino-4,5,6,7-tetrahydro-6-n-propylamino-benzothiazol, gegebenenfalls in Form seines (+) oder seines (-) Enantiomers, gegebenenfalls in Form der pharmakologisch verträgliche Säureadditionssalze sowie Hydrate und Solvate zur Herstellung eines Arzneimittels zur Vorbeugung und/oder Behandlung des Status epilepticus.

Zur Behandlung und/oder Vorbeugung in oben genannten medizinischen Indikationen kann neben einer Monotherapie mittels Pramipexol alternativ auch eine Kombinationstherapie von Pramipexol mit einer oder mehreren, bevorzugt einer anderen pharmzeutisch wirksamen Verbindung durchgeführt werden. Hierbei kann sich beispielsweise die Kombination mit weiteren Antikonvulsiva als besonders wirksam erweisen.

Pramipexol kann zur Behandlung der vorstehend genannten Zustände in Kombination, beispielsweise mit einem oder mehreren, bevorzugt einer der folgenden Substanzen sinnvoll zum Einsatz gelangen: Carbamazepin, Oxcarbamazepin, Valproinsäre, Diphenylhydantoin, Ethosuximid, Mesuximid, Phenobarbital, Primidon, Benzodiazepine (bevorzugt Diazepam, Clonazepam oder Clobazam), Corticotrophin, Corticoide, Bromide (beispielsweise Kaliumbromid), Sultiam, Acetazolamid, Felbamat, Gabapentin, Lamotrigen, Topiramat, Vigabatrin, Levetiracetam und Zonisamide.

Pramipexol kann im Rahmen der vorliegenden Erfindung als Racemat, in Form seines (+)- oder in Form seines (-)-Enantiomers zum Einsatz gelangen. Femer kann Pramipexol in Form seiner pharmazeutisch verträglichen Säureadditionssalze sowie gegebenenfalls in Form seiner Hydrate und/oder Solvate verwendet werden. Unter pharmazeutisch verträglichen Säureadditionssalzen werden erfindungsgemäß solche Salze verstanden, die ausgewählt sind aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure, wobei die Salze der Salzsäure, Bromwasserstoffsäure, Schwefelsäure, Phosphorsäure, und Essigsäure besonders bevorzugt sind. Eine besondere Bedeutung kommt hierbei den Salzen der Salzsäure zu. Besonders bevorzugt gelangen im Rahmen der vorliegenden Erfindung dementsprechend die Hydrochloride des Pramipexols zum Einsatz, wobei dem Pramipexoldihydrochlorid eine besondere Bedeutung zukommt. Von den Hydraten des Pramipexols ist das Pramipexoldihydrochlorid-monohydrat besonders bevorzugt.

Die Dosierung von Pramipexol ist naturgemäß stark abhängig vom Krankeitsbild. Beispielsweise und ohne den Gegenstand der vorliegenden Erfindung darauf zu beschränken kann Pramipexol pro Tag in Dosierungen von etwa 0,05 bis 7,5 mg, bevorzugt 0,1 bis 5 mg Verwendung finden. Diese Dosierungen sind bezogen auf Pramipexol in Form seiner freien Base. Bezogen auf die bevorzugt zum Einsatz gelangende Salzform Pramipexoldihydrochlorid-monohydrat entsprechen die vorstehend genannten Dosierungen etwa 0,07 bis 10,65 mg, bevorzugt 0,14 bis 7,1 mg Pramipexoldihydrochlorid-monohydrat pro Tag.

Eine mögliche und nur als beispielhaft erläuternd zu verstehende Vorgehensweise zur Dosierung ist nachfolgend bezogen auf Pramipexol in Form seiner freien Base ausgeführt: Individuelle Dosistitration in wöchentlichen Abständen je nach Wirkung und Verträglichkeit.
1. Woche: 3mal täglich 1 Tablette enthaltend 0,088 mg Pramipexol;
2. Woche: 3mal täglich 1 Tablette enthaltend 0,18 mg Pramipexol;
3. Woche und folgende: 3mal täglich 1/2 Tablette enthaltend 0,7 mg Pramipexol.
Je nach Stärke des Krankheitsbildes kann es gegebenenfalls auch erforderlich sein, Pramipexol in höheren Dosen zu applizieren. In solchen Fällen bieten sich Maximaldosierungen von etwa 5-7,5 mg Pramipexol pro Tag an.

Pramipexol kann im Rahmen der erfindungsgemäßen Anwendung oral, transdermal, intrathecal, inhalativ oder parenteral verabreicht werden. Geeignete Anwendungsformen sind beispielsweise Tabletten, Kapseln, Zäpfchen, Lösungen, Säfte, Emulsionen, dispersible Pulver oder Pflaster. Bezüglich möglicher Ausführungsformen einer erfindungsgemäß einsetzbaren transdermalen Applikationsform wird an dieser Stelle auf die Ausführungsbeispiele gemäß US 5112842 verwiesen, auf die hiermit ausdrücklich Bezug genommen wird. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Im Folgenden sind einige Beispiele für erfindungsgemäß einsetzbare pharmazeutische Zubereitungen angegeben. Diese dienen lediglich der beispielhaften Erläuterung, ohne den Gegenstand der Erfindung auf selbige zu beschränken.

### Tablette 1:

| Bestandteile: | mg |
|---|---|
| Pramipexoldihydrochlorid-monohydrat | 1,00 |
| Mannitol | 121,50 |
| Maisstärke | 79,85 |
| Hochdisperses Siliciumdioxid, wasserfrei | 2,30 |
| Polyvidon K25 | 2,35 |
| Magnesiumstearat | 3,00 |
| Gesamt | 210,00 |

### Tablette 2:

| Bestandteile: | mg |
|---|---|
| Pramipexol | 0,5 |
| Mannitol | 122,0 |
| Maisstärke, getrocknet | 61,8 |
| Maisstärke | 18,0 |
| Hochdisperses Siliciumdioxid, wasserfrei | 2,4 |
| Polyvidon K25 | 2,3 |
| Magnesiumstearat | 3,0 |
| gesamt | 210,0 |

### Tablette 3:

| Bestandteile: | mg |
|---|---|
| Pramipexol | 0,25 |
| Mannitol | 61,00 |
| Maisstärke | 39,90 |
| Hochdisperses Siliciumdioxid, wasserfrei | 1,20 |
| Polyvidon K25 | 1,15 |
| Magnesiumstearat | 1,5 |
| Gesamt | 105,00 |

### Tablette 4:

| Bestandteile: | mg |
|---|---|
| Pramipexol | 0,125 |
| Mannitol | 49,455 |
| Maisstärke getrocknet | 25,010 |
| Maisstärke | 7,300 |
| Hochdisperses Siliciumdioxid, wasserfrei | 0,940 |
| Polyvidon K25 | 0,940 |
| Magnesiumstearat | 1,230 |
| Gesamt | 85,000 |

### Lösung zur Injektion:

| | |
|---|---|
| Pramipexoldihydrochlorid-monohydrat | 0,3 mg |
| Natriumchlorid | 0,8 mg |
| Benzalkoniumchlorid | 0,01 mg |
| Aqua ad injectionem ad 100 ml | |

## Patentansprüche

1. Verwendung von 2-Amino-4,5,6,7-tetrahydro-6-n-propylamino-benzothiazol, gegebenenfalls in Form der pharmakologisch verträgliche Säureadditionssalze sowie Hydrate und Solvate zur Herstellung eines Arzneimittels mit antikonvulsiver Wirkung.

2. Verwendung nach Anspruch 1, zur Herstellung eines Arzneimittels zur Vorbeugung und/oder Behandlung cerebraler Anfälle.

3. Verwendung nach Anspruch 1 oder 2, **dadurch gekennzeichnet, daß** 2-Amino-4,5,6,7-tetrahydro-6-n-propylamino-benzothiazol in Form seines (+) oder seines (-) Enantiomers zum Einsatz kommt.

4. Verwendung nach einem der Ansprüche 1, 2 oder 3, zur Vorbeugung und/oder Behandlung des Status epilepticus, generalisierter Anfälle (Absencen, auch atypische Absencen, myoklonische, klonische, tonische und tonisch-klonische Anfälle), fokaler (einfach und komplex fokale) und sekundär generalisierter Anfälle.

5. Verwendung nach einem der Ansprüche 1, 2, 3 oder 4, zur Vorbeugung und/oder Behandlung des Status epilepticus.

6. Verwendung nach einem der Ansprüche 1 bis 5, **dadurch** gelennzeichnet, daß Pramipexol in Kombination mit einem oder mehreren weiteren Arzneistoffen zum Einsatz gelangt.

7. Verwendung nach Anspruch 6, **dadurch gekennzeichnet, daß** der Wirkstoff ausgewählt ist aus der Gruppe der Antikonvulsiva.

8. Verwendung nach Anspruch 6 oder 7, **dadurch gekennzeichnet, daß** Pramipexol mit einem oder mehreren Wirkstoffen ausgewählt aus der Gruppe bestehend aus Carbamazepin, Oxcarbamazepin, Valproinsäre, Diphenylhydantoin, Ethosuximid, Mesuximid, Phenobarbital, Primidon, Benzodiazepine, Corticotrophin, Corticoide, Bromide, Sultiam, Acetazolamid, Felbamat, Gabapentin, Lamotrigen, Topiramat, Vigabatrin, Levetiracetam und Zonisamide zum Einsatz gelangt.

9. Verwendung nach einem der Ansprüche 1 bis 8, **dadurch gekennzeichnet, daß** Pramipexol in Form eines pharmazeutisch verträglichen Säureadditionssalzes eingesetzt wird, welches ausgewählt ist aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure.

10. Verwendung nach einem der Ansprüche 1 bis 9, **dadurch gekennzeichnet, daß** Pramipexol pro Tag in Dosierungen von etwa 0,05 bis 7,5 mg, bevorzugt von etwa 0,1 bis 5 mg Verwendung findet.

## Claims

1. Use of 2-amino-4,5,6,7-tetrahydro-6-n-propylamino-benzothiazole, optionally in the form of the pharmacologically acceptable acid addition salts thereof as well as hydrates and solvates for preparing a pharmaceutical composition with an anticonvulsant activity.

2. Use according to claim 1, for preparing a pharmaceutical composition for the prevention and/or treatment of cerebral seizures.

3. Use according to claim 1 or 2, **characterised in that** 2-amino-4,5,6,7-tetrahydro-6-n-propylamino-benzothiazole is used in the form of the (+) or (-) enantiomer thereof.

4. Use according to one of claims 1, 2 or 3, for the prevention and/or treatment of epilepsy, generalised seizures (absences, also atypical absences, myoclonic, clonic, tonic and tonic-clonic seizures), focal (simple and complex focal) and secondary generalised seizures.

5. Use according to one of claims 1, 2, 3 or 4, for the prevention and/or treatment of epilepsy.

6. Use according to one of claims 1 to 5, **characterised in that** pramipexole is used in combination with one or more other pharmaceutical substances.

7. Use according to claim 6, **characterised in that** the active substance is selected from among the anticonvulsants.

8. Use according to claim 6 or 7, **characterised in that** pramipexole is used with one or more active substances selected from among carbamazepine, oxcarbamazepine, valproic acid, diphenylhydantoin, ethosuximide, mesuximide, phenobarbital, primidone, benzodiazepines, corticotrophin, corticoids, bromides, sultiam, acetazolamide, felbamat, gabapentin, lamotrigen, topiramat, vigabatrin, levetiracetam and zonisamide.

9. Use according to one of claims 1 to 8, **characterised in that** pramipexole is used in the form of a pharmaceutically acceptable acid addition salt which is selected from the salts of hydrochloric acid, hydrobromic acid, sulphuric acid, phosphoric acid, methanesulphonic acid, acetic acid, fumaric acid, succinic acid, lactic acid, citric acid, tartaric acid and maleic acid.

10. Use according to one of claims 1 to 9, **characterised in that** pramipexole is used in doses of about 0.05 to 7.5 mg, preferably about 0.1 to 5 mg per day.

## Revendications

1. Utilisation du 2-amino-4,5,6,7-tétrahydro-6-n-propylamino-benzothiazole, éventuellement sous forme des sels d'addition d'acide pharmacologiquement acceptables ainsi que des hydrates et solvates pour la production d'un médicament à activité anticonvulsivante.

2. Utilisation selon la revendication 1 pour la production d'un médicament pour la prévention et/ou le traitement des crises d'épilepsie partielles.

3. Utilisation selon la revendication 1 ou 2 **caractérisée en ce que** le 2-amino-4,5,6,7-tétrahydro-6-n-propylamino-benzothiazole est utilisé sous forme de son énantiomère (+) ou de son énantiomère (-).

4. Utilisation selon l'une des revendications 1, 2 ou 3 pour la prévention et/ou le traitement de l'état de mal épileptique, des crises généralisées (absences, également absences atypiques, crises myocloniques, cloniques, toniques et tonico-cloniques), des crises focales (focales simples et complexes) et généralisées secondaires.

5. Utilisation selon l'une des revendications 1, 2, 3 ou 4 pour la prévention et/ou le traitement de l'état de mal épileptique.

6. Utilisation selon l'une des revendications 1 à 5 **caractérisée en ce que** le pramipexol est utilisé en combinaison avec une ou plusieurs autres substances médicamenteuses.

7. Utilisation selon la revendication 6 **caractérisée en ce que** le principe actif est choisi dans le groupe des anticonvulsivants.

8. Utilisation selon la revendication 6 ou 7 **caractérisée en ce que** le pramipexol est utilisé avec un ou plusieurs principes actifs choisis dans le groupe consistant en carbamazépine, oxcarbamazépine, acide valproïque, diphénylhydantoïne, éthosuximide, mésuximide, phénobarbital, primidone, benzodiazépines, corticotrophine, corticoïdes, bromures, sultiame, acétazolamide, felbamate, gabapentine, lamotrigène, topiramate, vigabatrine, levetiracétame et zonisamide.

9. Utilisation selon l'une des revendications 1 à 8 **caractérisée en ce que** le pramipexol est utilisé sous forme d'un sel d'addition d'acide pharmaceutiquement acceptable qui est choisi parmi les sels de l'acide chlorhydrique, de l'acide bromhydrique, de l'acide sulfurique, de l'acide phosphorique, de l'acide méthanesulfonique, de l'acide acétique, de l'acide fumarique, de l'acide succinique, de l'acide lactique, de l'acide citrique, de l'acide tartrique et de l'acide maléique.

10. Utilisation selon l'une des revendications 1 à 9 **caractérisée en ce que** le pramipexol est utilisé par jour en des dosages d'environ 0,05 à 7,5 mg, de préférence d'environ 0,1 à 5 mg.
